# EUROPÄISCHE PATENTSCHRIFT

(11) **EP 1 874 692 B1**
(45) Veröffentlichungstag und Bekanntmachung des Hinweises auf die Patenterteilung: **11.05.2016**
(21) Anmeldenummer: 06754841.2
(22) Anmeldetag: 25.04.2006
(51) Int. Cl.: C02F 1/467, B41F 33/00

(54) **VERFAHREN UND VORRICHTUNG ZUR KONTROLLE UND BEGRENZUNG DER VERKEIMUNG INFEUCHTMITTELKREISLÄUFEN**
PROCESS AND DEVICE FOR THE CONTROL OF MICROBIAL CONTAMINATION IN WATER CIRCUITS
PROCÉDÉ ET DISPOSITIF DE CONTRÔLE DE LA CONTAMINATION MICROBIENNE DANS LES CIRCUITS D'EAU

(30) Priorität: 26.04.2005 EP 05103372
(43) Veröffentlichungstag der Anmeldung: 09.01.2008
(73) Patentinhaber: Felix Böttcher GmbH & Co. KG, 50933 Köln (DE)
(72) Erfinder: SANDER, Helmut, 50374 Erftstadt (DE)
(74) Vertreter: von Kreisler Selting Werner - Partnerschaft von Patentanwälten und Rechtsanwälten mbB
(86) Internationale Anmeldenummer: PCT/EP2006/061817
(87) Internationale Veröffentlichungsnummer: WO 2006/114414

(56) Entgegenhaltungen:
- EP-A- 0 647 594
- EP-A- 1 505 038
- DE-A1- 10 014 289
- DE-A1- 19 503 191
- DE-U1- 29 816 175
- US-A- 5 772 859
- US-B1- 6 324 974
- PATERMARAKIS G ET AL: "DISINFECTION OF WATER BY ELECTROCHEMICAL TREATMENT" WATER RESEARCH, PERGAMON PRESS, OXFORD, GB, Bd. 24, Nr. 12, 1. Dezember 1990 (1990-12-01), Seiten 1491-1496, XP000175524 ISSN: 0043-1354

## Beschreibung

Die vorliegende Erfindung betrifft ein Verfahren und eine Vorrichtung zur Kontrolle und Begrenzung der Verkeimung in Feuchtmittelkreisläufen.

Feuchtmittel (Feuchtwasser) wird eingesetzt um hydrophile Bereiche von Druckplatten an den Stellen, die keine Druckfarbe aufnehmen sollen, hydrophil zu halten.

Das Feuchtwasser wird über das Feuchtwerk direkt oder indirekt auf die Druckplatte aufgetragen, die Druckfarbe nimmt dabei eine begrenzte Menge Feuchtmittel auf und bildet eine Emulsion. Das Feuchtwasser enthält Komponenten, die die nichtdruckenden Partien der Druckplatte hydrophil halten, dadurch wird die Annahme von Druckfarbe in diesem Bereich verhindert.

Zum Einsatz kommt dies insbesondere bei Flachdruckverfahren, bei denen die druckenden und nicht-druckenden Stellen der Druckform in nahezu einer Ebene liegen. Diese Druckverfahren beruhen auf dem gegensätzlichen physikalischchemischen Verhalten bestimmter Substanzen in der Beschichtung der Druckplatte, die farbannehmend oder farbabstoßend sind.

Im Offsetdruck sind dies eine dünne Druckplatte (z.B. aus Aluminium), auf deren Oberfläche sich druckfarbannehmende (lipophile) Bildstellen und bildfreie (hydrophile) Stellen befinden.

Größenordnungsmäßig verbraucht eine Druckmaschine zwischen 10 und 150 Liter Feuchtwasser pro Stunde. Dabei wird das Feuchtwasser typischerweise leicht gekühlt verwendet.

Feuchtmittel enthält neben Wasser typischerweise pH-regulierende Substanzen (Puffer), Konservierungsmittel (z.B. Biozide), Korrosionsinhibitoren, Plattenschutzkomponenten, sowie gegebenenfalls Netzmittel. Diese Komponenten sind üblicherweise in einem Feuchtmittelkonzentrat vorhanden und werden dann mit Wasser auf eine vorgegebene Konzentration verdünnt. Das gebrauchsfertige Feuchtmittel wird im Kreislauf geführt. Die einzelnen Feuchtwerke der Druckmaschine sind über eine Zirkulationsleitung mit der Feuchtmittelversorgungsanlage verbunden.

In der Vorlaufleitung wird das Feuchtmittel dem Feuchtwerk zugeführt. In der Rücklaufleitung fließt das überschüssige, mit Farb- und Papierpartikeln verschmutzte Feuchtmittel wieder zur Aufbereitungsanlage zurück. Je nach Ausführung der Anlage wird das zurückfließende Feuchtmittel über Filtermaterial oder Filterbeutel aus Kunstfasern von Schmutzpartikeln gereinigt, bevor es in das Auffangbecken der Anlage fließt. An diesen Feuchtmittelaufbereitungsgeräten befindet sich auch üblicherweise das Kühlgerät, mit dem die Feuchtmitteltemperatur konstant auf einen vorgegebenen Wert gehalten wird, der typischerweise im Bereich von 9 bis 15°C liegt.

Während des Druckprozesses wird ständig Feuchtmittel verbraucht und durch Zudosieren von frischem Feuchtmittel ergänzt. Trotz Kühlung und Filtration kommt es immer wieder vor, dass die Keimbelastung im Feuchtmittelkreislauf stark ansteigt. Die Schmutzpartikel aus dem Druckprozess, insbesondere Cellulose aus dem Papierstrich, aber auch die verwendeten Puffer, sorgen für günstige Wachstumsbedingungen für die Keime.

Es kommt zur Ausbildung von Biofilmen in den Rohrleitungen, Filtern, Feuchtwerken und im Becken der Feuchtmittelversorgungsanlage. Bei zunehmender Verkeimung werden Puffersubstanzen abgebaut und der pH-Wert steigt an, was negative Folgen im Druckprozess hat, beispielsweise schlechteres Anlaufverhalten, erhöhte Wasserführung und instabiles Farb-Wassergleichgewicht.

Die Verkeimung im Feuchtmittelkreislauf lässt sich im Druckbetrieb nur ungenügend durch die Zugabe von Bioziden verhindern; Keime werden durch die Biozide nur im Wachstum gehemmt, nicht abgetötet. Auswahl und Konzentration dieser Produkte ist wegen der gesundheitlichen Gefährdung der an den Druckmaschinen arbeitenden Personen begrenzt.

Bei einer übermäßigen Verkeimung des Feuchtmittelkreislaufes muss das gesamte System gereinigt und mit stark alkalischen Lösungen (beispielsweise in Kombination mit Wasserstoffperoxyd, Hypochlorit oder anderen hochwirksamen Bakteriziden) 5 bis 8 Stunden gespült werden. Solche Reinigungszyklen sind typischerweise alle ein bis zwei Monate erforderlich.

Das Problem der Verkeimung im Feuchtmittelkreislauf ist umso stärker, je größer die Druckmaschinen und damit verbunden die Kreislaufsysteme der Rohrleitungen sind. Das gesamte, mit hoher Durchflussgeschwindigkeit im Kreislauf zirkulierte Flüssigkeitsvolumen liegt bei Zeitungsdruckmaschinen häufig im Bereich von 200 bis 500 Litern, die über große Distanzen transportiert werden müssen. Besonders anfällig für Verkeimungen sind Zeitungsmaschinen, da hier die Rohrleitungen einen größeren Durchmesser haben und daher nie vollständig mit Feuchtmittel gefüllt sind. Hinzu kommt der noch verstärkte Eintrag von Papierstaub, der zum Großteil aus Cellulose besteht.

In Lösung freilebende Bakterien sind durch Biozide oder Antibiotika relativ leicht angreifbar und kontrollierbar. Haben sich erst einmal Biofilme gebildet, sind diese wesentlich resistenter. In den Biofilmen haften die Bakterien aneinander oder am Untergrund, dabei sind sie von extrazellulären Substanzen umgeben, die hauptsächlich aus Polysachariden und Proteinen bestehen. Diese Schleimschicht schützt die Bakterien sehr wirksam vor äußeren Einflüssen. Mit den handelsüblichen Bioziden lassen sich die Biofilme in den Feuchtwasserleitungssystemen der Druckmaschinen nicht sicher bekämpfen. Haben sich erst einmal Keimbeläge im Feuchtsystem gebildet, wachsen sie sehr schnell weiter und können den Druckbetrieb empfindlich stören. Abgelöste Biofilme und Agglomerate können in die Feuchtwerke gespült werden, wo sich Filter, Düsen und Ventile zusetzen. Im Feuchtmittelaufbereitungssystem macht sich ein vermehrtes Keimwachstum durch einen fauligen Geruch im Feuchtmittelbecken bemerkbar. Auf den Filtermatten des Rücklaufes und an den Wandungen des Feuchtmittelbeckens bildet sich eine Schleimschicht aus.

EP 0 647 594 A2 beschreibt einen elektrostatischen Wassersterilisierapparat.
DE 298 16 175 U1 beschreibt eine Wasserbehandlungseinrichtung, die ionisches Wasser erzeugen kann.
US 5,772,859 beschreibt ein Verfahren zur Behandlung von Feuchtmittel in einer Druckpresse, bei dem durch eine Behandlung mit Gleichstrom die Trocknung der Tinte verbessert wird.

US 6,324,974 B1 beschreibt ein Verfahren zur Vermeidung der Keimbildung in Feuchtmittelkreisläufen unter Verwendung eines elektromagnetischen Feldes.

DE 100 14 289 beschreibt ein Verfahren zur Desinfektion und Sterilisierung unter Verwendung von elektrischen Strömen und Feldern.
Aufgabe der vorliegenden Erfindung war es daher, ein Verfahren und eine Vorrichtung zu entwickeln, mit der der Verkeimung von Feuchtmittelkreisläufen entgegen gewirkt werden kann.
Gelöst wird die Aufgabe durch ein Verfahren umfassend den Schritt der elektrochemischen Behandlung des Feuchtmittels gemäß Anspruch 1. Erfindungsgemäß erfolgt dies durch das Anlegen einer Spannung zwischen mindestens zwei Elektroden im Feuchtmittelkreislauf.
Das Verfahren dient der Verminderung des Keimwachstums im Feuchtmittelkreislauf. Hierdurch kann sowohl die Neubildung gehemmt oder verhindert werden, als auch bei vorhandener Verkeimung eine Reduktion der Keimzahl erreicht werden.
Die Spannung ist eine Wechselspannung. Das Umpolen vermeidet die Entstehung von Spaltprodukten durch Elektrolyse, sowie die Bildung von Belägen auf den Elektrodenoberflächen. Besonders geeignet ist eine Rechteckspannung als Wechselspannungsform. Die Frequenz der Wechselspannung liegt im Bereich von 0,01 bis 20.000 Hz, bevorzugt 0,05 bis 10 oder 1 bis 10 Hz.
Bei Einsatz von Wechselspannung liegt die Spannung im Bereich von 5 bis 50 Volt, bevorzugt im Bereich von 5 bis 20 Volt. Da sehr hohe Spannungen Gefahren für das Bedienungspersonal aufweisen, werden niedrigere Spannungswerte bevorzugt.

Die mindestens zwei Elektroden können an jeder geeigneten Stelle des Feuchtmittelkreislaufes angebracht werden, beispielsweise im Feuchtmittelzulauf, im Zirkulationssystem, z.B. integriert ins Rohrleitungssystem oder im Feuchtmittelbecken. Die elektrochemische Behandlung des Feuchtmittels über die Elektroden kann auch von einem externen Gerät aus erfolgen. Dabei wird im Bypass ein Teil des Feuchtmittels in diesem Gerät behandelt und dann dem Kreislauf wieder zugeführt. Es ist grundsätzlich auch möglich, mehr als zwei Elektroden oder zwei oder mehr Elektrodenpaare einzusetzen, die an verschiedenen Stellen angebracht sind.

Geeignete Materialien für die Elektroden können aus Metallen, Halbleitern, leitfähiger Keramik, Graphit oder leitfähigen Kunststoffen ausgewählt werden. Besonders bevorzugte Materialien für die Elektroden sind Titan, Titanlegierungen oder beschichtetes Titan. Beschichtungen mit Platin, Iridium, Indium, Rutheniumoxid, Indiumoxid, Iridiumoxid oder Mischungen hiervon sind besonders bevorzugt.

Es wird vermutet, dass sich bei der Behandlung Sauerstoffradikale bilden, die sehr viel stärker auf Biofilme einwirken, als zum Beispiel Chlor.

Die Kontrolle der Verkeimung umfasst die Verhinderung des Wachstums und das Abtöten der Keime.

Es wird auch eine Klärung des Feuchtmittels beobachtet. Das im Laufe der Betriebszeit eher trübe und eingefärbte Feuchtmittel wird während der Behandlung zunehmend klarer.

Die Behandlung kann kontinuierlich erfolgen. Sie kann aber auch tageweise oder stundenweise unterbrochen werden. Der Betrieb kann insbesondere in Abhängigkeit vom Keimwachstum gesteuert werden.

Gegenstand der Erfindung ist auch eine Vorrichtung zur Durchführung des Verfahrens, die ein Feuchtmittelsystem mit mindestens zwei Elektroden und eine Wechselspannungsquelle umfasst. Ein Feuchtmittelsystem umfasst Feuchtwerke, Zirkulationsleitungen und eine Feuchtmittelversorgungsanlage. Diese Feuchtmittelversorgungsanlage umfasst ein Feuchtmittelaufbereitungsgerät, ein Kühlgerät, eine Dosiereinheit und ein Feuchtmittelbecken. Das Feuchtmittelaufbereitungsgerät umfasst Pumpeneinheiten für die Zirkulation und die Filtereinrichtungen.

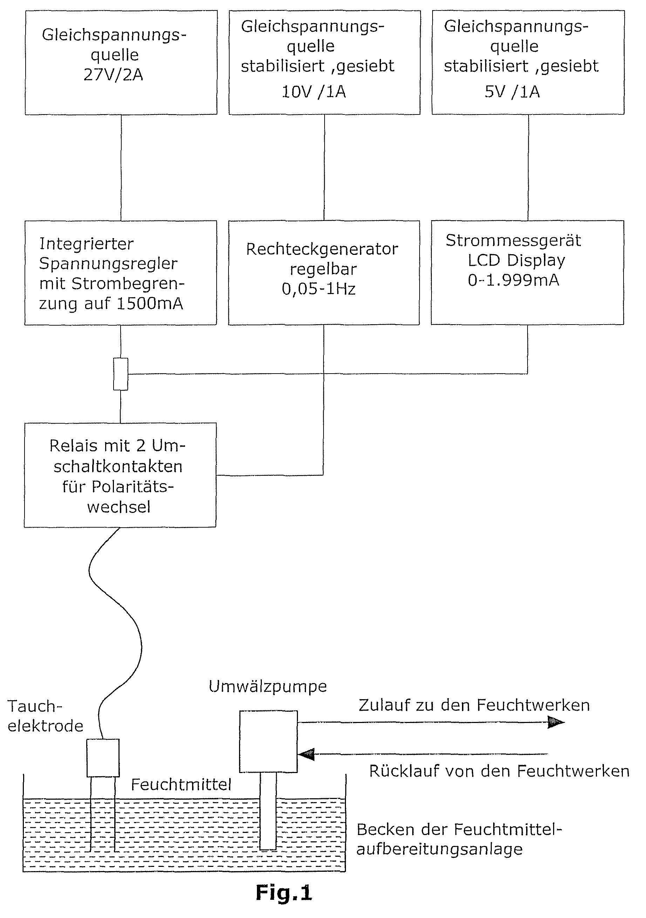

Figur 1 zeigt schematisch eine Ausführungsform der elektrochemischen Behandlungsvorrichtung.

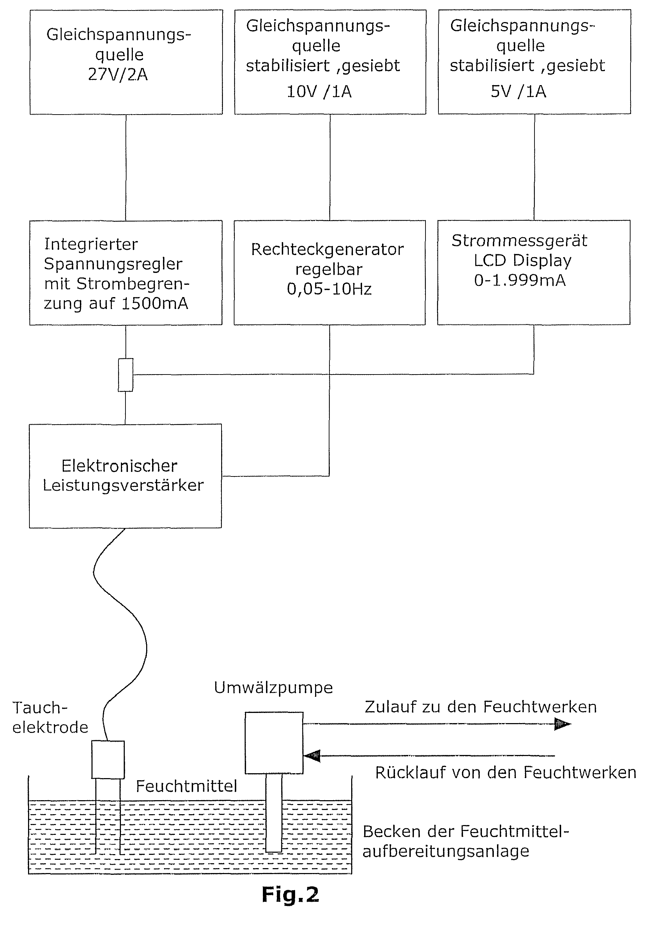

Figur 2 zeigt eine alternative Ausführungsform der elektrochemischen Behandlungsvorrichtung.

Die Wirkungsweise des Systems wird durch das folgende Beispiel näher erläutert. Der Aufbau der Anlage ist wie folgt.

Der benötigte Strom dieser Anlage erfolgt über einen Transformator mit einer primären Eingangsspannung aus dem Netz von 230 bis 240 V und Wechselstromausgängen von 25 V, 12 V und 6 V. Der Transformator hat eine Leistung von 100 W.

Aus dem 25 V Wechselstromausgang wird über einen Brückengleichrichter und anschließende Siebung mit einem 2200 µF Elektrolytkondensator eine Gleichspannung von 28 V erzeugt.

Um die Anlage vor Überlastungen und Kurzschlüssen zu schützen, wird der Ausgangsstrom über einen regelbaren Spannungsregler, der in Reihe geschaltet ist, auf 1,5 A begrenzt. Bei Überlastung erwärmt sich der Spannungsregler und regelt den Strom automatisch herunter.

Aus dem 12 V Wechselstromausgang wird über einen Brückengleichrichter eine Gleichspannung von 14 V erzeugt und durch einen integrierten Spannungsregler auf 10 V stabilisiert. Die Siebung erfolgt durch einen 1000 µF Elektrolytkondensator.

Mit der stabilisierten 10 V Gleichspannung wird der Rechteckgenerator versorgt.

Die Bauteile im Rechteckgenerator sind so dimensioniert, dass das Verhältnis von Pause und Impuls 1:1 ist. Der Rechteckgenerator ist über ein Potentiometer im Bereich von 0,05 bis 10 Hz regelbar.

An dem Ausgang des Rechteckgenerators ist ein Relais angeschlossen. Über zwei Umschaltkontakte wird die Polarität der 28 V Gleichspannung (Punkt 2) abhängig von der Frequenz des Rechteckgenerators kontinuierlich gewechselt, dadurch wird eine Rechteck-Wechselspannung erzeugt.

Der Relaisausgang ist mit der Tauchelektrode verbunden. Diese besteht aus zwei Titanblechen mit den Abmessungen 300 x 30 mm und einer Stärke von 3 mm. Die elektrischen Anschlüsse sind zum Schutz mit Epoxydharz eingegossen. Die Elektrodenplatten haben einen Abstand von 10 mm zueinander.

Aus dem 6 V Wechselstromausgang wird über einen Brückengleichrichter eine Gleichspannung von 8 V erzeugt und durch einen integrierten Spannungsregler auf 5 V stabilisiert. Die Siebung erfolgt durch einen 1000 µF Elektrolytkondensator.

Mit der stabilisierten 5 V Gleichspannung wird das digitale Anzeigedisplay für die Strommessung versorgt.

Der Strom wird über den Spannungsabfall an einem 1 Ohm Widerstand, der in Reihe zwischen dem Strombegrenzer und dem Relais liegt, gemessen. Der Messbereich geht von 0 bis 1,999 mA.

Im Testversuch wurde ermittelt, dass beim Einschalten des Gerätes der Stromfluss zunächst stark ansteigt und dann nach 1 bis 2 Stunden abfällt und sich stabilisiert. Der Stromzufluss hängt sehr stark ab von der Leitfähigkeit des Feuchtmittels. Bevorzugt liegt der Stromfluss in der Größenordnung von 0,1 bis 2 A, bevorzugt 0,1 bis 0,6 A.

Nach zwei bis vier Tagen lösten sich verstärkt Biofilme und andere Schmutzagglomerate aus den Rohrleitungen und dem Umlaufsystem ab, wurden ausgespült und sammelten sich im Rücklauf auf der Filtermatte. Weiterhin konnte man beobachten, wie das Feuchtwasser von Tag zu Tag klarer wurde.

Die Keimbelastung wurde mit sogenannten DIP-Slides untersucht. Eingesetzt wurden hierzu Envirocheck Contact Slides der Firma MERCK, Darmstadt, Deutschland. Es handelt sich dabei um Agar-beschichtete Platten (Caseinpepton-Sojamehlpepton-Agar). Die Rückseite der DIP slides enthält die gleiche Beschichtung mit dem Zusatz eines Enthemmers, mit dem die Wirkung der Biozide im Feuchtmittel neutralisiert wird. Die DIP slides wurden drei Tage bei 36 °C bebrütet und ausgewertet.

Die Anzahl der gebildeten Kolonien (Kolonie-bildende Einheiten) ist ein Maß für die Keimbelastung im Feuchtmittel (KBE/ml). Es wurden sowohl Abstriche des Filters als auch des Feuchtmittels untersucht. Weitere Untersuchungen erfolgten 24 bzw. 48 Stunden nach Beginn der elektrochemischen Wasserbehandlung.

| Dip Slides | | ohne elektrochemischer Wasserbehandlung | mit elektrochemischer Wasserbehandlung | |
|---|---|---|---|---|
| | | | nach 24h | nach 48h |
| Seite 1 KBE/ml | Filter | 10⁶-10⁷ | 10⁵ | 10³-10⁴ |
| | Feuchtmittels | 10⁶ | 10³-10⁴ | 10² |
| Seite 2 KBE/ml mit Enthemmer | Filter | > 10⁷ | 10⁵-10⁶ | 10⁴ |
| | Feuchtmittels | 10⁶-10⁷ | 10⁴ | 10²-10³ |

Wie ersichtlich, nimmt die Keimbelastung nach 24 Stunden der erfindungsgemäßen Behandlung um mehrere Zehnerpotenzen ab und erreicht damit einen Wert, der im Feuchtwasserkreislauf unkritisch ist. Das erfindungsgemäße Verfahren hatte keine Auswirkungen auf den Druckprozess und auf die Eigenschaften des Feuchtwassers, wie z. B. pH-Wert oder Leitfähigkeit.

## Patentansprüche

1. Verfahren zur Reduzierung der Verkeimung von Kreisläufen mit wässrigen Medien umfassend den Schritt der elektrochemischen Behandlung des wässrigen Mediums, wobei die elektrochemische Behandlung durch das Anlegen einer Spannung an mindestens zwei Elektroden erfolgt, der Kreislauf ein Feuchtmittelkreislauf ist, **dadurch gekennzeichnet, dass** die Spannung eine Wechselspannung ist, die Spannung im Bereich von 5 bis 50 Volt liegt und die Frequenz der Wechselspannung im Bereich von 0,01 bis 20.000 Hertz liegt.

2. Verfahren nach Anspruch 1, **dadurch gekennzeichnet, dass** die Wechselspannung eine Rechteckspannung, Sinusspannung oder Dreieckspannung ist.

3. Verfahren nach Anspruch 1 oder 2, **dadurch gekennzeichnet, dass** die Frequenz der Wechselspannung im Bereich von 1 bis 20.000 Hertz liegt.

4. Verfahren nach mindestens einem der Ansprüche 1 bis 3, **dadurch gekennzeichnet, dass** die Spannung im Bereich bis 25 Volt liegt.

5. Verfahren nach mindestens einem der Ansprüche 1 bis 4, **dadurch gekennzeichnet, dass** die mindestes zwei Elektroden im Feuchtmittelzulauf, im Zirkulationssystem oder in einem Bypass angebracht sind.

6. Verfahren nach mindestens einem der Ansprüche 1 bis 5, **dadurch gekennzeichnet, dass** das Material für die mindestens zwei Elektroden unabhängig voneinander aus Metallen, Halbleitern, leitfähiger Keramik, Graphit oder leitfähigen Kunststoffen ausgewählt wird.

7. Verfahren nach mindestens einem der Ansprüche 1 bis 6, **dadurch gekennzeichnet, dass** das Material für die mindestens zwei Elektroden unabhängig voneinander aus Titan, Titanlegierungen oder beschichtetem Titan ausgewählt werden.

8. Verfahren nach Anspruch 7, **dadurch gekennzeichnet, dass** die Beschichtung des Titans mit Platin, Iridium, Indium, Rutheniumoxid, Indiumoxid, Iridiumoxid oder Mischungen davon erfolgt.

9. Vorrichtung zur Durchführung des Verfahrens nach mindestens einem der Ansprüche 1 bis 8 umfassend ein Kreislaufsystem, mindestens zwei Elektroden und eine Spannungsquelle, wobei das Kreislaufsystem ein Feuchtmittelkreislauf ist und die Spannungsquelle eine Wechselspannungsquelle ist.

## Claims

1. A process for reducing the microbial contamination of circulations with aqueous media, comprising the step of electrochemically treating the aqueous medium, wherein the electrochemical treatment is effected by applying a voltage to at least two electrodes, said circulation is a fountain solution circulation, **characterized in that** said voltage is an alternating voltage, said voltage is within a range of from 5 to 50 volts, and the frequency of said alternating voltage is within a range of from 0.01 to 20,000 Hertz.

2. The process according to claim 1, **characterized in that** said alternating voltage is a square-wave voltage, a sinusoidal wave voltage, or a triangular wave voltage.

3. The process according to claim 1 or 2, **characterized in that** the frequency of said alternating voltage is within a range of from 1 to 20,000 Hertz.

4. The process according to at least one of claims 1 to 3, **characterized in that** said voltage is within a range of up to 25 volts.

5. The process according to at least one of claims 1 to 4, **characterized in that** said at least two electrodes are positioned in the fountain solution supply, in the circulation system, or in a bypass.

6. The process according to at least one of claims 1 to 5, **characterized in that** the material for said at least two electrodes is independently selected from metals, semiconductors, conductive ceramics, graphite or conductive plastics.

7. The process according to at least one of claims 1 to 6, **characterized in that** the material for said at least two electrodes is independently selected from titanium, titanium alloys or coated titanium.

8. The process according to claim 7, **characterized in that** the coating of said coated titanium is effected using platinum, iridium, indium, ruthenium oxide, indium oxide, iridium oxide or mixtures thereof.

9. A device for performing the process according to at least one of claims 1 to 8, comprising a circulation system, at least two electrodes and a voltage source, wherein said circulation system is a fountain solution circulation and said voltage source is an alternating voltage source.

## Revendications

1. Procédé destiné à la réduction de la prolifération des germes dans des circuits avec des milieux aqueux, comprenant l'étape de traitement électrochimique du milieu aqueux, le traitement électrochimique étant réalisé par l'application d'une tension sur au moins deux électrodes, le circuit étant un circuit de solution de mouillage, **caractérisé en ce que** la tension est une tension alternative, la tension se situe dans une plage comprise entre 5 et 50 volts et la fréquence de la tension alternative se situe dans une plage comprise entre 0,01 et 20000 hertz.

2. Procédé selon la revendication 1, **caractérisé en ce que** la tension alternative est une tension rectangulaire, une tension sinusoïdale ou une tension triangulaire.

3. Procédé selon la revendication 1 ou 2, **caractérisé en ce que** la fréquence de la tension alternative se situe dans une plage comprise entre 1 et 20000 hertz.

4. Procédé selon au moins l'une des revendications 1 à 3, **caractérisé en ce que** la tension se situe dans une plage comprise jusqu'à 25 volts.

5. Procédé selon au moins l'une des revendications 1 à 4, **caractérisé en ce que** les au moins deux électrodes sont positionnées dans l'alimentation de la solution de mouillage, dans le système de circulation ou dans un conduit de dérivation.

6. Procédé selon au moins l'une des revendications 1 à 5, **caractérisé en ce que** le matériau pour les au moins deux électrodes est sélectionné, indépendamment l'une de l'autre, parmi les métaux, les semi-conducteurs, la céramique conductrice, le graphite ou les plastiques conducteurs.

7. Procédé selon au moins l'une des revendications 1 à 6, **caractérisé en ce que** le matériau pour les au moins deux électrodes est sélectionné, indépendamment l'une de l'autre, parmi le titane, des alliages en titane ou de titane revêtu.

8. Procédé selon la revendication 7, **caractérisé en ce que** le revêtement du titane est réalisé avec du platine, de l'iridium, de l'indium, de l'oxyde de ruthénium, de l'oxyde d'indium, de l'oxyde d'iridium ou leurs mélanges.

9. Dispositif destiné à la réalisation du procédé selon au moins l'une des revendications 1 à 8, comprenant un système de circuit, au moins deux électrodes et une source de tension, le système de circuit étant un circuit de solution de mouillage et la source de tension étant une source de tension alternative.
